(19)

Europäisches Patentamt

European Patent Office

Office européen des brevets

(11) **EP 3 572 825 A2**

(12) **EUROPEAN PATENT APPLICATION**

(43) Date of publication:
**27.11.2019 Bulletin 2019/48**

(51) Int Cl.:
**G01R 33/56** *(2006.01)*  **G01R 33/561** *(2006.01)*

(21) Application number: **19174901.9**

(22) Date of filing: **16.05.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **24.05.2018  US 201862675893 P**

(71) Applicant: **Siemens Healthcare GmbH**
**91052 Erlangen (DE)**

(72) Inventors:
 • **Körzdörfer, Gregor**
  **91058 Erlangen (DE)**
 • **Nittka, Mathias**
  **91083 Baiersdorf (DE)**
 • **Pang, Jianing**
  **Chicago, IL 60646 (US)**
 • **Speier, Peter**
  **91056 Erlangen (DE)**

(54) **DICTIONARY COMPRESSION FOR MAGNETIC RESONANCE FINGERPRINTING**

(57)    A method, MR system and storage medium providing a first magnetic resonance fingerprinting dictionary, the fingerprints of the magnetic resonance fingerprinting dictionary having a first length,
providing at least one transformation matrix, the transformation matrix at least being designed to shorten the fingerprints to a second length shorter than the first length,
obtaining a second magnetic resonance fingerprinting dictionary by multiplying the first magnetic resonance fingerprinting dictionary with the transformation matrix, the fingerprints of the magnetic resonance fingerprinting dictionary having the second length.

FIG 2

EP 3 572 825 A2

## Description

BACKGROUND OF THE INVENTION

Field of the Invention

The present invention concerns methods for reducing artifacts in parameter maps created by magnetic resonance fingerprinting ("MRF").

Description of the Prior Art

A basic problem for acquiring magnetic resonance ("MR") images is the scan time. This time was initially reduced by software methods in the form of optimized pulse sequences, wherein the flip angle of the pulses, the number thereof, the setting of the gradients or the waiting times between individual sequence sections, was modified. It was thus possible to reduce the acquisition of a gradient echo image using the FLASH method from several minutes to a few seconds. Although this changes the contrast behavior, it remains $T_2^*$-dependent. The RARE method is well-known as a fast, spin echo based imaging method. Other methods such as GRASE or TrueFISP exist, which involve combinations of the basic methods.

In order to achieve a further reduction in the acquisition time, it has been proposed to use multiple coils for reading out the scan signal. Here, not all the k-space lines are acquired; rather only selected selected k-space lines are acquired using the multiple coils. This is also known as undersampling. In order to prevent an aliasing artefact, i.e. foldover effects, which appear in the reconstructed image due to this procedure, different reconstruction algorithms are used that require fewer k-space lines and therefore the more time-consuming scanning (filling) of the k-space lines is unnecessary.

Such reconstruction methods are commonly referred to under the acronyms GRAPPA (GeneRalized Autocalibrating Partially Parallel Acquisition), SENSE (SENSitivity Encoding for fast MRI) and SMASH (SiMultaneous Acquisition of Spatial Harmonics).

Even if these methods have reduced the scan time enormously, the acquisition of a parameter map like a $T_1$-map remains time consuming. Therefore, usually, conventional images are acquired having a known weight with regard to physical parameters. For example, a FLASH sequence having a long echo time TE produces $T_2^*$-weighted images.

These qualitative images can be interpreted by a radiologist or generally a physician for specific disease signatures. Such an interpretation requires a lot of experience but stays highly subjective due to the fact that only signal differences are examined.

In contrast to this, with the use of quantitative MR imaging techniques, absolute properties of the scanned examination object may be determined in humans, for example in particular the tissue-specific T1 and T2 relaxation. The property can be represented, for example, as parameter maps, which reproduce the parameter values such as the respective relaxation times, in a spatially resolved manner. Quantitative techniques therefore provide the advantage of an objective comparability, but, due to long scanning times, are currently rarely used as a matter of routine.

With the use of newer scanning methods, such as magnetic resonance fingerprinting methods (MRF methods), the drawback of long scanning times can be reduced to an acceptable extent. The principle of these methods is to generate, by pseudo random variation of sequence parameters (flip angle, TR, gradient,...), well differentiable signal characteristics for various substances. Different relaxation parameters of a substance yield differing signal evolutions in response to the excitation pattern. The acquired signal evolutions are compared with a large number of simulated signal evolutions, which were created in advance. A single simulated signal evolution is called a "fingerprint". The totality of the fingerprints is called a "dictionary". The fingerprints are simulated by varying the desired parameters, i.e. $T_1$, $T_2$, $B_0$, $B_1$, or ADC, which is the apparent diffusion coefficient. The process of comparing measured and simulated signal evolutions and finding the best match is called "matching". The fingerprint characteristic which is most similar to the scanned one determines the relaxation parameters, for example $T_1$ and $T_2$, of the pixel in question, which, in turn, allows conclusions to be made as to the tissue from which the MR signal originated in order to create that pixel's signal.

Magnetic resonance fingerprinting methods are described, for example, in the article by Ma et al., "Magnetic Resonance Fingerprinting", Nature, 495: p. 187-192 (2013).

To generate the signal evolutions, up to 3000 images are acquired. In this case every fingerprint has 3000 complex data points. If the desired parameters that span the dictionaries' non-time dimensions are varied in reasonable steps, the dictionary can easily contain tens of thousands of fingerprints even if only three parameters are varied. Then the dictionary comprises several Gigabyte of data. With four parameters, it can already be in the order of Terabytes. Additionally, the time for matching rises.

To reduce the processing time, it is known to compress the dictionary. This is described in the article by McGivney et al., "SVD Compression for Magnetic Resonance Fingerprinting in the Time Domain", IEEE Transactions on Medical Imaging, 33 (12) : p. 2311-2322 (2014).

There the uncompressed dictionary D, which is a matrix, is factorized according to the singular value decomposition:

$$D = U\Sigma V^*$$

where $U \in C^{pxp}$ and $V \in C^{qxq}$ are unitary matrices, and $\Sigma \in \mathbb{R}^{pxq}$ is a diagonal matrix containing the non-increasing singular values $\sigma_i$; i = 1, ... , min{p, q}.

The dictionary D is then approximated by a low-rank approximation

$$D \approx U_k \Sigma_k V_k^*$$

where k < r, r = rank(D).

The compressed dictionary $D_k$ is calculated by multiplying the uncompressed dictionary with the so called compression matrix $V_k$:

$$D_k = V_k^* D$$

After the described operations one has the matrix $\Sigma_k$ containing the singular values, the compression matrix $V_k$ with the basis vectors and the compressed dictionary $D_k$ containing the coefficients for the basis vectors.

To match an acquired signal evolution with the content of the compressed dictionary $D_k$, the signal evolution x also has to be compressed:

$$x_k = V_k^* x$$

The compressed vector $x_k$ also contains coefficients for the basis vectors of the compression matrix $V_k$. Then the matching process can be executed by comparing the coefficients of $x_k$ with those of $D_k$.

This process reduces disk usage and processing time simultaneously.

Performing full template matching between acquired signals and a compressed MRF dictionary may be computationally less intensive compared to a conventional MRF dictionary containing all time points. Despite these efforts, MRF reconstruction continues to be a challenge, because the compressed dictionary only fits for a defined length of fingerprints given in advance.

## SUMMARY OF THE INVENTION

An object of the invention is to be flexible with regard to the number of images used for creating a signal evolution.

In accordance with one aspect of the invention, a method is provided for creating a dictionary corresponding to the length of a currently acquired signal evolution, the method including the following method steps: First, providing a first magnetic resonance fingerprinting dictionary, the fingerprints of the magnetic resonance fingerprinting dictionary having a first length. The first magnetic resonance fingerprinting dictionary contains, as explained above, information on simulated signal evolutions, the so-called fingerprints. The fingerprints have a first length which is the maximum length for simulated signal evolutions available. Therefore the first length is in the time domain and one of the dimensions of the uncompressed first magnetic resonance dictionary.

Second, providing at least one transformation matrix, the transformation matrix at least being designed to shorten the fingerprints to a second length shorter than the first length. The transformation matrix can fulfill several steps at the same time. It must at least shorten the fingerprints. Then the second length is always shorter than the first length. Doing so one has to provide only one dictionary that is always available on a storage medium, and a transformation matrix for every additional dictionary to be created. The transformation matrices are smaller in size than a complete dictionary and therefore storage space can be reduced. The second length also is in the time domain and one of the dimensions of the second magnetic resonance fingerprinting dictionary.

Third, obtaining a second magnetic resonance fingerprinting dictionary by multiplying the first magnetic resonance fingerprinting dictionary with the transformation matrix, the fingerprints of the magnetic resonance fingerprinting dictionary having the second length. After creating the second magnetic resonance fingerprinting dictionary the parameters associated with a signal evolution can be determined.

Let the first length be 3000. Here complex data points are always meant. The first dictionary can be used to determine parameters acquired by using a MRF pulse sequence that creates 3000 images. Of course the simulation algorithm to create the fingerprints of the first dictionary is based on the scan parameters of the pulse sequence.

If the number of images is reduced to e.g. 2000, the matching procedure obviously will fail. The first dictionary cannot be used for determination of the parameters of the signal evolutions.

One solution is to provide a second dictionary, the fingerprints of which have the right length, in this case 2000. To save storage space it is possible to provide the first magnetic resonance fingerprinting dictionary and a transformation matrix transforming the first magnetic resonance dictionary into a second magnetic resonance fingerprinting dictionary having the desired shorter length, in this case 2000 complex data points for every fingerprint.

Since theoretically 2999 additional dictionaries could be necessary it is clear that the usage of transformation matrices helps saving storage space for every length of fingerprint made available.

The second dimension of the first and second magnetic resonance fingerprinting dictionary is usually given by the number of fingerprints and keeps unchanged during the shortening process.

Accordingly, it is now possible to store only one first magnetic resonance fingerprinting dictionary having a first length, which is the maximum length available. Such first dictionary may be transformed to a second dictionary of a desired shorter length (second length), by the transformation matrix, which may be calculated in advance and provided on the MRI system. According to useful embodiments, a number of transformation matrices are provided, which are designed to shorten the fingerprints to a number of different second lengths, i.e. for various second lengths.

The first magnetic resonance fingerprinting dictionary may be stored in a compressed state, e.g. it may have been compressed by an SVD compression. For example, it has been compressed by a compression matrix adapted to the first length. This has the advantage that first dictionary requires even less storage space.

According to a useful embodiment, the transformation matrix is further designed to decompress the first magnetic resonance fingerprinting dictionary, especially if the first magnetic resonance fingerprinting dictionary is in a compressed state. Decompression may be done by multiplication of the adjunct of the compression matrix adapted to the first length. By further multiplication with the compression matrix for a shorter (second) length, one receives a compressed second magnetic resonance imaging fingerprinting dictionary having a shorter (second) length. Accordingly, in a useful embodiment, the transformation matrix may be the product of a decompression matrix and a compression matrix, e.g. the adjunct of the compression matrix adapted to the first length and a compression matrix for a shorter (second) length. Thereby, storage space can be saved.

According to a useful embodiment, the transformation matrix is further designed to compress the second magnetic resonance fingerprinting dictionary, in particular as explained above. Again, this saves storage space. Usually, such compression is associated with information loss, since this will result in effective data compression.

According to a useful embodiment, the second magnetic resonance fingerprinting dictionary is compressed to a rank lower than the rank of the first magnetic resonance fingerprinting dictionary.

The invention is also directed to a set of transformation matrices for shortening the fingerprints of a first magnetic resonance fingerprinting dictionary to a set of second lengths shorter than the first length, as described herein.

In accordance with another aspect of the invention a magnetic resonance imaging (MRI) system is disclosed comprising a magnet system configured to generate a polarizing magnetic field about at least a portion of a subject arranged in the MRI system; a plurality of gradient coils configured to apply a gradient field to the polarizing magnetic field; a radio frequency (RF) system configured to apply an excitation field to a subject and acquire MR image data from a region of interest (ROI); a storage medium providing a first magnetic resonance fingerprinting dictionary and a transformation matrix, the transformation matrix being designed to transform the first magnetic resonance fingerprinting dictionary to a second magnetic resonance fingerprinting dictionary, the second magnetic resonance fingerprinting dictionary being smaller than the first magnetic resonance fingerprinting dictionary and corresponding to the length of measured signal evolutions; a computer system programmed to: control the plurality of gradient coils and the RF system to acquire magnetic resonance fingerprinting (MRF) data from a subject; reconstruct an MRF time series of images from the MRF data; obtain signal evolutions out of the time series of images; obtain the second magnetic resonance fingerprinting dictionary; obtain MR parameters by matching the signal evolutions with the second magnetic resonance fingerprinting dictionary; generate MR parameter maps from the matched parameters.

The MRI system differs from known systems in providing a transformation matrix that can transform the first magnetic resonance fingerprinting dictionary to a second magnetic resonance fingerprinting dictionary being smaller and having a shorter length, respectively.

The invention is also directed to a processor being able to execute the method according to the invention. Such processor may be part of a workstation for controlling an MRI system, but may also be part of any standalone computer, PC, cloud computer, server, client-server architecture or any handheld device such as a tablet computer or mobile phone, or a portable device such as a laptop.

In accordance with another aspect of the invention a non-transitory computer-readable storage medium is provided, the storage medium storing computer executable instructions that when executed by a computer control the computer to perform a method for creating a dictionary corresponding to a desired length, in particular the length of a currently acquired signal evolution, the method including the following method steps:

providing a first magnetic resonance fingerprinting dictionary, the fingerprints of the magnetic resonance fingerprinting dictionary having a first length, providing at least one transformation matrix, the transformation matrix at least being designed to shorten the fingerprints to a second length shorter than the first length,
obtaining a second magnetic resonance fingerprinting dictionary by multiplying the first magnetic resonance fingerprinting dictionary with the transformation matrix, the fingerprints of the magnetic resonance fingerprinting dictionary having the second length.

In accordance with another aspect of the invention, a computer program having software code portions is provided, the software code portions causing a processor to perform the method of the invention when executed on a processor.

All advantages, features and embodiments of the invention explained with regard to one aspect of the invention are applicable to any other aspect of the invention.

BRIEF DESCRIPTION OF THE DRAWINGS

The foregoing and other objects, aspects, features, and advantages of the disclosure will become more apparent and better understood by referring to the following description taken in conjunction with the accompanying drawings, in which:

Fig. 1 illustrates a MRI system.
Fig. 2 illustrates a method for generating parameter maps using MRF.
Fig. 3 illustrates an example method of comparing a signal evolution to fingerprints.
Fig. 4 illustrates an example method.
Fig. 5 illustrates an example method of generating a second magnetic resonance fingerprinting dictionary.
Fig. 6 illustrates an example method of generating a second magnetic resonance fingerprinting dictionary.
Fig. 7 illustrates an example method of generating a second magnetic resonance fingerprinting dictionary.
Fig. 8 illustrates a flip angle distribution for an MRF pulse sequence.
Fig. 9 illustrates a flip angle distribution for an MRF pulse sequence.
Fig. 10 illustrates a storage medium.

DETAILED DESCRIPTION OF THE INVENTION

Exemplary embodiments of an apparatus, storage mediums and methods as described here serve to improve conventional magnetic resonance fingerprinting (MRF) by providing a transformation matrix for a magnetic resonance fingerprinting dictionary to adapt the dictionary to current scan parameters. MRF acquires and processes magnetic resonance (MR) data to simultaneously provide quantitative maps of different tissue parameters through pattern recognition. A predefined magnetic resonance fingerprinting dictionary may model the possible signal evolutions simulated, by using, for example, Bloch equations with different combinations of various MR parameters. The MR parameters may be one or more out of the group $T_1$, $T_2$, $T_2^*$, $B_0$, $B_1$, ADC, $M_0$. Pattern recognition may be completed by computing the inner product between the noisy signal and predicted signals in the dictionary. The dictionary may also store previously observed signal evolutions.

Example systems, storage mediums and methods apply a compression to the first magnetic resonance fingerprinting dictionary. Reducing the size of the MRF dictionary in the time domain yields faster reconstruction of the tissue parameters (e.g., $T_1$, $T_2$, off-resonance) without sacrificing the accuracy of MRF.

The example systems, storage mediums and methods apply SVD as a compression method to the first magnetic resonance fingerprinting dictionary. Usage of singular value decomposition allows a compression without information loss if applied properly.

The example systems, storage mediums and methods may comprise a decompression matrix to decompress the first magnetic resonance fingerprinting dictionary. The compressed first magnetic resonance dictionary $Dc_1$ can be decompressed to the uncompressed dictionary $D_1$ by multiplying the decompression matrix $V_k^{-1}$ on it (wherein the original dictionary may not exactly, but approximately reproduced):

$$D_1 \approx V_K^{-1} * Dc_1$$

To clarify detailed examples having concrete values are given. Let n be the number of time points of a fingerprint, m the number of fingerprints and 1 the number of data points after compression. Some example values are m = 5000, n = 3000 and 1 = 200. Then the following is given: $D_1 \epsilon \mathbb{C}^{3000x5000}$, $Dc_1 \epsilon \mathbb{C}^{200x5000}$ and $V_k^{-1} \epsilon \mathbb{C}^{3000x200}$.

The index 1 denotes the first magnetic resonance fingerprinting dictionary. The uncompressed dictionary has the letter D, the compressed dictionary the letters Dc. This also holds for the second dictionary.

The second magnetic resonance fingerprinting dictionary may be achieved by a multiplication of the first magnetic resonance fingerprinting dictionary $D_1$ with the transformation matrix $T_{jxn}$:

$$D_2 \approx T_{j \times n} * D_1$$

The transformation matrix $T_{j \times n} \epsilon \mathbb{C}^{jxn}$ only shortens the first magnetic resonance fingerprinting dictionary $D_1$ which contains fingerprints having a first length. The second dimension is the number of fingerprints m, which here remains unchanged. The first length is given by n. The dictionary is shortened to j data points. Let j be 2000, then $T_{j \times n} \epsilon \mathbb{C}^{2000x3000}$ and $D_2 \epsilon \mathbb{C}^{2000x5000}$. Here the fingerprints have the length j, which is the second length. That means shortening the first magnetic resonance fingerprinting dictionary $D_1$ in the time dimension from m to j. The number of fingerprints m remains unchanged.

The example system and methods may comprise the transformation matrix being designed to decompress the first magnetic resonance fingerprinting dictionary. The transformation matrix is then a combination of the steps of decompression and shortening and is denoted $T_{j \times l}$:

$$D_2 \approx T_{j \times l} * Dc_1$$

The transformation matrix $T_{j \times l} \in \mathbb{C}^{j \times l}$ decompresses and shortens the first magnetic resonance fingerprinting dictionary $Dc_1$, which contains coefficients for the basis vectors of the matrix $V_k$. The transformation matrix $T_{j \times l}$ can be achieved by a combination of the shortening matrix $T_{j \times n}$ and the decompression matrix $V_k^{-1}$ :

$$T_{j \times l} = T_{j \times n} * V_k^{-1}$$

The example systems, storage mediums and methods may comprise the transformation matrix T being designed to compress the second magnetic resonance fingerprinting dictionary $Dc_2$. If the first magnetic resonance fingerprinting dictionary $D_1$ is not compressed the transformation matrix $T_{i \times n}$ shortens the first magnetic resonance fingerprinting dictionary $D_1$ and compresses the second magnetic resonance fingerprinting dictionary $D_2$ to a compressed matrix $Dc_2$ having size i in the compressed dimension:

$$Dc_2 = T_{i \times n} * D_1$$

where the transformation matrix $T_{i \times n}$ is given by:

$$T_{i \times n} = V_{i \times j} * T_{j \times n}$$

Hence the transformation matrix $T_{i \times n} \in \mathbb{C}^{i \times n}$ is a product of a compression matrix $V_{i \times j} \in \mathbb{C}^{i \times j}$ that compresses a matrix in one dimension from j to i. The notation says that the matrix $T_{i \times n}$ is of dimension i x n and has complex data points.

The example systems, storage mediums and methods may comprise the second magnetic resonance fingerprinting dictionary $Dc_2$ being compressed to a rank $kc_2$ lower than the rank $k_1$ of the first magnetic resonance fingerprinting dictionary $D_1$, in particular lower than the rank $kc_1$ of the compressed first magnetic resonance fingerprinting dictionary $D_1$. The rank $kc_2$ may be so small that a loss of information occurs. This is negligible because the second magnetic resonance fingerprinting dictionary $Dc_2$ is not processed further. On the contrary this accelerates the matching process. Then i may be set to 20.

Therefore, the size of the compressed magnetic resonance fingerprinting dictionary $Dc_2$ may be less than 25% of the size of the first magnetic resonance fingerprinting dictionary $Dc_1$. In an embodiment, the size of the compressed second magnetic resonance fingerprinting dictionary $Dc_2$ is less than 10% of the size of the compressed first magnetic resonance fingerprinting dictionary $Dc_1$.

The example system and methods may apply SVD as compression method to the second magnetic resonance fingerprinting dictionary $Dc_2$.

The example systems, storage mediums and methods may comprise the transformation matrix being designed to decompress the first magnetic resonance fingerprinting dictionary $Dc_1$, shorten the decompressed first magnetic resonance fingerprinting dictionary $D_1$ and compress the second magnetic resonance fingerprinting dictionary $Dc_2$. Then the transformation matrix is a combination of the steps of decompression, shortening compression and is denoted $T_{i \times l}$:

$$Dc_2 \approx T_{i \times l} * Dc_1$$

where the transformation matrix $T_{i \times l} \in \mathbb{C}^{i \times l}$ is given by:

$$T_{i \times l} = V_{i \times j} * T_{j \times n} * V_{n \times l}^{-1}.$$

Here the transformation matrix $T_{i \times l}$ is achieved by a combination of the compression matrix $V_{i \times j} \in \mathbb{C}^{i \times j}$, the shortening matrix $T_{j \times n}$ and the decompression matrix $V_{n \times l}^{-1} \in \mathbb{C}^{n \times l}$. The dimensions are given by $T_{i \times l} \in \mathbb{C}^{20 \times 200}$, $Dc_2 \in \mathbb{C}^{20 \times 5000}$, $V_{n \times l}^{-1} \in \mathbb{C}^{3000 \times 200}$, $T_{j \times n} \in \mathbb{C}^{2000 \times 3000}$ and $V_{i \times j} \in \mathbb{C}^{20 \times 2000}$.

The example systems, storage mediums and methods may comprise the signal evolution being compressed before matching. If the second dictionary $Dc_2$ is compressed then the signal evolution y also has to be compressed to the compressed signal evolution yc:

$$yc = V_{i \times j} * y$$

The matching works then as follows:
The signal evolution yc has the second length and is smaller than the first length. After the compression the vector yc has the coefficients belonging to the matrix $V_{ixj}$ which includes the basis vectors. Here $y \epsilon \mathbb{C}^{j \times 1}$ and $yc \epsilon \mathbb{C}^{i \times 1}$. This describes y being a one-column vector having j data points and yc being a one-column vector having i complex data points.

The example systems, storage mediums and methods may comprise the calculation and storing of the first magnetic resonance fingerprinting dictionary $D_1$ or $Dc_1$ and the transformation matrices T before executing the MRF pulse sequence generating the raw data. In particular they may be stored before delivering the MR system.

The example systems, storage mediums and methods may comprise the first magnetic resonance fingerprinting dictionary and the second magnetic resonance fingerprinting dictionary having two dimensions, one of them being in the time domain and the shortening procedure carried out in the time domain.

The example systems, storage mediums and methods may comprise the first magnetic resonance fingerprinting dictionary and the second magnetic resonance fingerprinting dictionary having two dimensions, one of them being in the time domain and the compression carried out in the time domain.

The example systems, storage mediums and methods may comprise the first magnetic resonance fingerprinting dictionary and the second magnetic resonance fingerprinting dictionary having two dimensions, one of them being the number of fingerprints.

This may be explained in detail with the accompanying drawings:

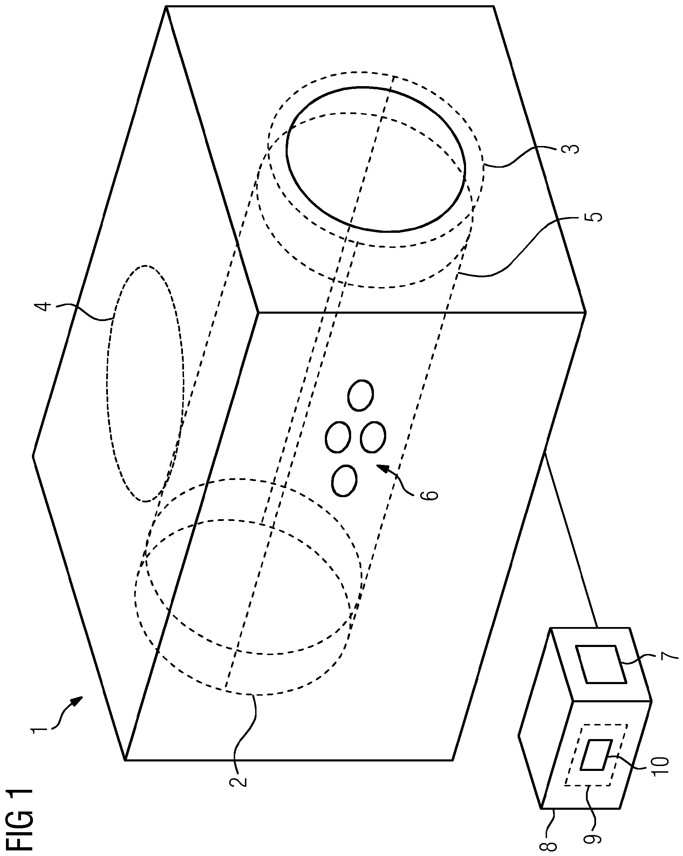

FIG. 1 shows a magnetic resonance system 1 having a magnet system. The MR system 1 also includes three gradient coils 2, 3 and 4 configured to apply a gradient field to the polarizing magnetic field. To apply an excitation field to a subject and acquire MR image data from a ROI a radio frequency (RF) system having two RF coils 5 and 6 is provided.

The RF coil 5 is implemented as an excitation coil and the RF coil 6 as a detection coil. The RF coil 6 is normally adapted to specific sections of the patient, e.g. as a "head coil", "chest coil" or "knee coil". The RF coil 5 is also known as a "body coil" and is less sensitive than the RF coil 6, but homogeneous over a larger area. The RF coil 6 may be designed as coil array allowing parallel imaging.

The gradient coils 2, 3 and 4 produce gradient fields in mutually orthogonal directions. In order to produce a resulting gradient in a predefined direction, the slice direction, read direction or phase encoding direction, the gradient fields of two gradient coils or all three gradient coils 2, 3 and 4 can also be superimposed. A gradient is therefore identical to the gradient field of a single gradient coil only in exceptional cases, and is usually a superposition of a number of gradient fields.

Furthermore, the MR system 1 provides a storage medium 7. The storage medium 7 stores a first magnetic resonance fingerprinting dictionary and a transformation matrix, the transformation matrix being designed to transform the first magnetic resonance fingerprinting dictionary to a second magnetic resonance fingerprinting dictionary. The storage medium 7 is described in detail later.

The first magnetic resonance fingerprinting dictionary may be an uncompressed first magnetic resonance fingerprinting dictionary $D_1$ or a compressed first magnetic resonance fingerprinting dictionary $Dc_1$. The transformation matrix T may be designed as a shortening matrix $T_{jxn}$, a decompressing and shortening matrix $T_{jxl}$, a shortening and compressing matrix $T_{ixn}$ or a decompressing, shortening and compressing matrix $T_{ixl}$.

The fingerprints of the first magnetic resonance fingerprinting dictionary $D_1$ have a first length n, the number of the coefficients may have a number of 1.

To cover signal evolutions being shorter than the first length n the transformation matrix T is used. Depending on the compression state of the first and second magnetic resonance fingerprinting directory one of the transformation matrices $T_{jxn}$, $T_{ixn}$, $T_{jxl}$ or $T_{ixl}$ is used.

Additionally the MR system 1 has a computer system 8 having a processor 9 programmed to control the plurality of gradient coils 2, 3, 4 and the RF system including the RF coils 5 and 6, to acquire magnetic resonance fingerprinting (MRF) data from a subject; to reconstruct an MRF time series of images from the MRF data; to obtain signal evolutions out of the time series of images; to obtain the second magnetic resonance fingerprinting dictionary $D_2$; to obtain MR parameters by matching the signal evolutions with the second magnetic resonance fingerprinting dictionary; and to generate MR parameter maps from the matched parameters.

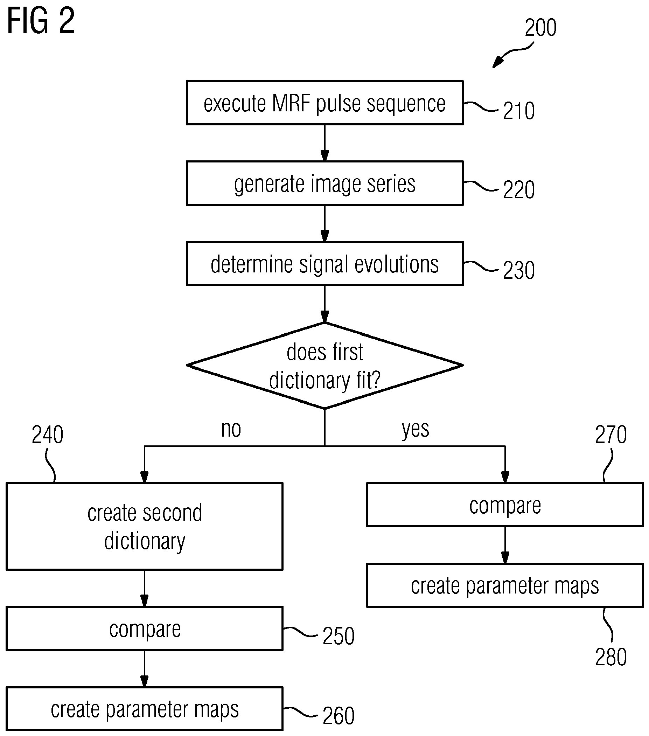

FIG. 2 illustrates an example method 200 for generating a parameter map using magnetic resonance fingerprinting. At 210 a MRF pulse sequence is executed. The MRF pulse sequence is designed to acquire raw data. During application of the pulse sequence at least one scan parameter, e.g. echo time TE, flip angle $\alpha$, or repetition time TR is varied to create a defined change in the acquired signal over time. The raw data comprises or consists of information to generate a certain number of images, which is done at 220. Also the number of images is a scan parameter which can be changed.

After having reconstructed the images, the signal evolutions are determined out of the images at 230. A signal evolution is received by adding the signal amplitudes of a given pixel for every image of the image series to a vector, which is then the signal evolution of this pixel.

Method 200 also includes, at 240, upon determining that the length of the first magnetic resonance fingerprinting dictionary does not correspond to the length of the image series acquired at 210, the creation of a second magnetic resonance dictionary using a transformation matrix T. The various embodiments to generate the second magnetic resonance fingerprinting dictionary are described in detail later.

The the signal evolutions are then compared to the entries of the dictionary at 250. This step may include a compression of the signal evolution if the dictionary is compressed. Then for every signal evolution a set of parameters like $T_1$, $T_2$, and so on are available.

These parameters are used to create parameter maps for every parameter at 260.

If the first magnetic resonance fingerprinting dictionary fits, then the length of the signal evolutions the steps of matching and creation of parameter maps can be done at 270 and 280 without generating a second magnetic resonance fingerprinting dictionary.

The step of determining whether the length of the first magnetic resonance dictionary fits is not necessarily done directly before the matching process. In fact, it can be performed at any time after the number of images to be acquired is fixed. Then the generation of the second magnetic resonance fingerprinting dictionary can be done parallel to the acquisition of the raw data or during the reconstruction of the image series.

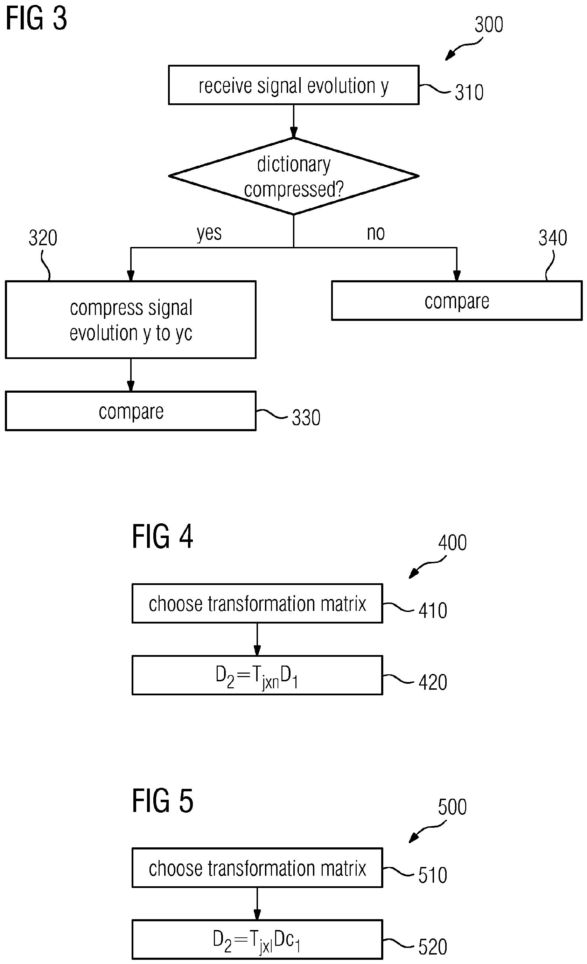

FIG. 3 illustrates an example method 300 of comparing a signal evolution to the dictionary entries which may take place during portion 250 or 270 of method 200.

At 310 a signal evolution y is received. Then it has to be determined, if the dictionary in use, whether the first magnetic resonance fingerprinting dictionary or the second magnetic resonance fingerprinting directory, is a compressed dictionary.

In case of a compressed dictionary the signal evolution is also compressed at 320. This is executed by using the same compression matrix $V_{ixj}$ that was used to compress the dictionary $Dc_2$. After the compression of the signal evolution y the comparison can be done at 330. Here the coefficients are compared within the SVD domain.

Otherwise the comparison can be done at once at 340. This comparison takes place in the time domain.

FIG. 4 illustrates an example method 400 of creating a second magnetic resonance fingerprinting dictionary $D_2$ in a first embodiment, which may take place during portion 240 of method 200.

It should be mentioned that there will be more than one transformations matrix T, independent of the following embodiments. The first magnetic resonance fingerprinting dictionary $D_1$ defines the maximum number of images which is equivalent to the length of the signal evolutions.

To recover a dictionary corresponding to shorter lengths, a transformation matrix is needed for every length to be generated. The number of transformation matrices can be limited by allowing a restricted possible number of images. The length of the signal evolutions then also only has a limited number of possible values.

For example, the number of images may only be a multiple of 100. Then the number of images to be acquired may be 100, 200, 300, ..., 3000. Then only 30 transformation matrices T are necessary, one to shorten the first magnetic resonance fingerprinting dictionary to a length of 100 data points, one to shorten the first magnetic resonance fingerprinting dictionary to a length of 200 data points, and so on.

At 410 the transformation matrix $T_{jxn}$ having a suitable length with regard to the length of the signal evolution is chosen.

The uncompressed first magnetic resonance dictionary $D_1$ is then multiplied with the transformation matrix $T_{jxn}$ at 420 to receive the uncompressed second magnetic resonance dictionary $D_2$.

The matching process can then be executed in the time domain. After usage the second magnetic resonance dictionary $D_2$ can be deleted again.

The embodiment according to FIG. 4 saves storage space, because the transformation matrix $T_{jxn}$ is smaller than the second magnetic resonance dictionary $D_2$.

FIG. 5 illustrates an example method 500 of creating a second magnetic resonance fingerprinting dictionary $D_2$ in a second embodiment, which may take place during portion 240 of method 200.

At 510 the transformation matrix $T_{jxl}$, having a suitable length with regard to the length j of the signal evolution, is chosen. As mentioned several times before a transformation matrix has to be stored for every length j to be generated.

In the second embodiment the first magnetic resonance fingerprinting dictionary $Dc_1$ is compressed. Hence the transformation matrix $T_{jxl}$ decompresses the compressed first magnetic resonance dictionary $Dc_1$ and shortens it to the not compressed second magnetic resonance fingerprinting dictionary $D_2$ at 520:

$$D_2 = T_{j \times l} * Dc_1 .$$

The first magnetic resonance fingerprinting dictionary $Dc_1$ is stored permanently; therefore, a minimization of the used disk space is desired.

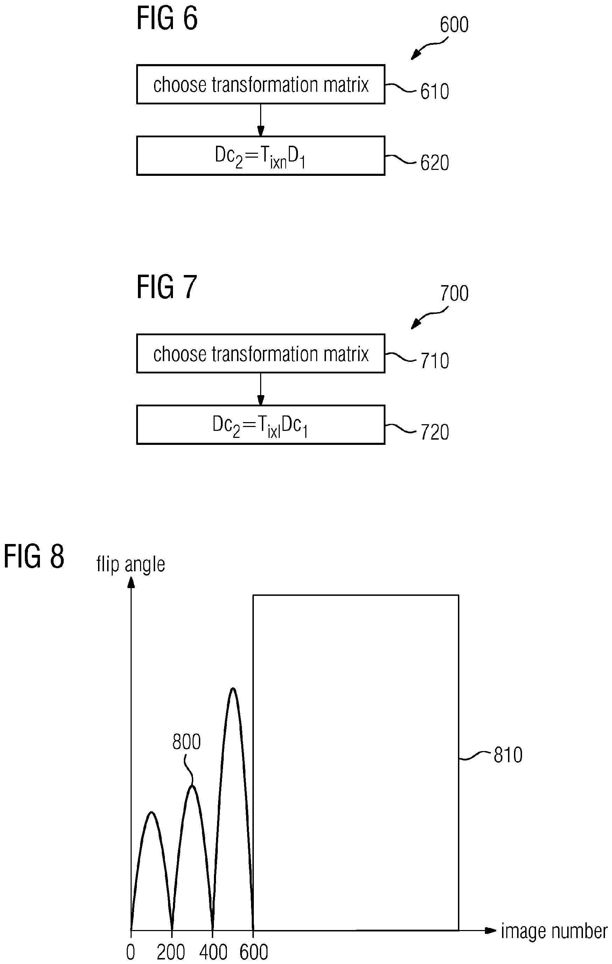

FIG. 6 illustrates an example method 600 of creating a compressed second magnetic resonance fingerprinting dictionary $Dc_2$ in a third embodiment which may take place during portion 240 of method 200.

At 610 the transformation matrix $T_{ixn}$ having a suitable length with regard to the length of the signal evolution is chosen.

In the second embodiment the first magnetic resonance fingerprinting dictionary $D_1$ is not compressed. Hence the transformation matrix $T_{ixn}$ shortens the first magnetic resonance dictionary $D_1$ and compresses it to the length i of the compressed second magnetic resonance fingerprinting dictionary $Dc_2$ at 620:

$$Dc_2 = T_{i \times n} * D_1 .$$

The compression of the second magnetic resonance fingerprinting dictionary $Dc_2$ can be carried out with or without signal loss. The compression to a rank with a significant information loss is preferred for two reasons:

First the second magnetic resonance fingerprinting dictionary $Dc_2$ may be used more often and stored for a longer time. Since a dictionary $Dc_2$ is necessary for every second length j several dictionaries $Dc_2$ may be stored. Second the matching process is the faster the smaller the second magnetic resonance fingerprinting dictionary $Dc_2$ is. In other words, the speed of the matching process corresponds inversely to the size of the second MRF dictionary $Dc_2$.

FIG. 7 illustrates an exemplary method 700 for creating a compressed second magnetic resonance fingerprinting dictionary $Dc_2$ in a fourth embodiment which may take place during portion 240 of method 200.

At 710 the transformation matrix $T_{ixl}$ having a suitable length with regard to the length of the signal evolution is chosen.

In the fourth embodiment the first magnetic resonance fingerprinting dictionary $Dc_1$ is compressed. Hence the transformation matrix $T_{ixl}$ decompresses the compressed first magnetic resonance dictionary $Dc_1$, shortens it and compresses it to the length i of the the compressed second magnetic resonance fingerprinting dictionary $Dc_2$ at 720:

$$Dc_2 = T_{i \times l} * Dc_1$$

Here, all benefits of compression are implemented. The fourth embodiment is the best mode example with regard to the generation of the second magnetic resonance fingerprinting dictionary $Dc_2$.

FIG. 8 shows a possible flip angle distribution used to create six hundred images. FIG. 8 shows the flip angle curve 800 with respect to image number. To create the images a FISP sequence with spiral readout can be used. One excitation pulse is then executed and a total image can be acquired in one shot. Each flip angle then stands for one image.

If the first magnetic resonance fingerprinting dictionary $Dc_1$ is designed for a certain number of for example 3000 images, which is the first length n, then the transformation matrix shortens it to 600 data points, which is the second length j, according to the number of images acquired. As described above the signal evolutions are achieved from the 600 images pixel-wise.

This is shown by the area 810 which hides the elements removed by the transformation matrix $T_{ixl}$.

The first magnetic resonance fingerprinting dictionary $Dc_1$ and the second magnetic resonance finger-printing dictionary $Dc_2$ may be compressed.

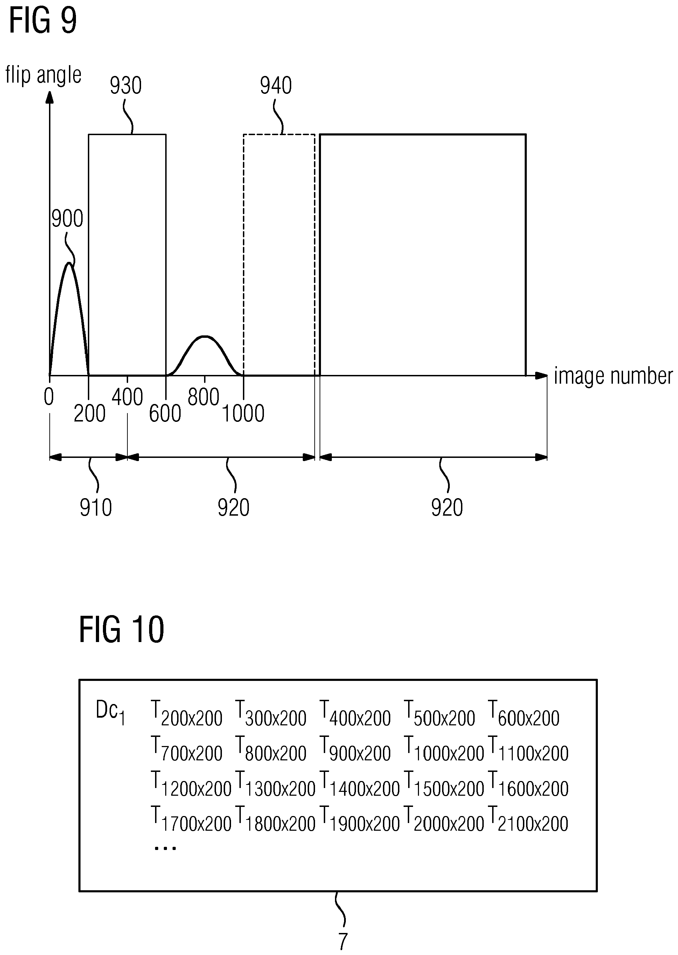

FIG. 9 shows a result of a shortening when the MRF pulse sequence includes different pulse sequences. The curve 900 indicates again the flip angle. The first two hundred images in the first block 910 are acquired using a FISP sequence. Following these are four hundred images acquired with a FLASH sequence in a second block 920. The first magnetic resonance fingerprinting dictionary $Dc_1$ is designed for six hundred images in the first block 910 and eight hundred images in the second block 920. The first block 910 and the second block 920 may be followed by further blocks so that 3000 images may be acquired in total.

The transformation matrix $T_{ixl}$ then removes data points in the middle and at the end of the fingerprints. This is indicated by the masking areas 930 and 940.

It should be noted that the curves 800 and 900 do not show signal evolutions or fingerprints; rather, they show flip angle curves. Every flip angle belongs to an image and every image adds a data point to a signal evolution. Therefore a flip angle also stands for a data point of a signal evolution even if the signal evolution may have a completely different shape.

FIG. 10 shows the storage medium 7 introduced in FIG. 1 in more detail. It can be seen that a compressed first magnetic resonance fingerprinting dictionary $Dc_1$ is stored. Its dimensions are l x m and for example $Dc_1 \epsilon \mathbb{C}^{200 x 5000}$. Hence there are 5000 fingerprints having a length of 200 data points in the SVD domain. The first length n cannot be seen directly. Additionally a plurality of transformation matrices can be found. They are of dimension $T_{j \times l} \epsilon \mathbb{C}^{j \times l}$ to distinguish clearly between the different transformation matrices for different second lengths j. The length 1 is always the same and matches the length 1 of the first dictionary $Dc_1$. These transformation matrices can be used to generate second magnetic resonance fingerprinting dictionaries $D_2$ with dimensions j x m, here j x 5000.

Obviously instead of translation matrices $T_{j \times l} \epsilon \mathbb{C}^{j \times l}$ there could be a plurality of translations matrices $T_{i \times l} \epsilon \mathbb{C}^{i \times l}$ stored. Using the index i there could be different translation matrices $T_{i \times l}$ having the same indices i and 1. This can happen because the matrices are given by

$$T_{i \times l} = V_{i \times j} * T_{j \times n} * V_{n \times l}^{-1},$$

see above. Then they can differ because they were generated using different matrices even if the indices i and 1 are the same.

The example values of i, j, 1, m, and n are only for explanation purposes and shall not limit the invention to these values.

If no transformation matrix of a length corresponding to the length of the acquired signal evolutions can be found on the storage medium or the MR system, the transformation matrix can be calculated with regard to the used MRF pulse sequence. This can be done either on the MR system or outside, e.g. on a large-capacity computer.

**Claims**

1. A method comprising:

    providing a first magnetic resonance fingerprinting dictionary ($DC_1$), the fingerprints of the magnetic resonance fingerprinting dictionary having a first length,
    providing at least one transformation matrix ($T_{ixl}$), the transformation matrix at least being designed to shorten the fingerprints to a second length shorter than the first length,
    obtaining a second magnetic resonance fingerprinting dictionary ($D_2$), by multiplying the first magnetic resonance fingerprinting dictionary with the transformation matrix($T_{ixl}$), the fingerprints of the magnetic resonance fingerprinting dictionary having the second length.

2. The method of claim 1, wherein the first magnetic resonance fingerprinting dictionary is compressed.

3. The method of one of the preceding claims, further comprising the transformation matrix ($T_{ixl}$) being designed to decompress the first magnetic resonance fingerprinting dictionary.

4. The method of one of the preceding claims, further comprising the transformation matrix ($T_{ixl}$) being designed to compress the second magnetic resonance fingerprinting dictionary.

5. The method of one of the preceding claims, wherein the second magnetic resonance fingerprinting dictionary is used for matching a signal evolution having the second length.

6. The method of claim 5, wherein the signal evolution is compressed before matching.

7. The method of one of the preceding claims, wherein the transformation matrix is chosen out of a plurality of transformation matrices ($T_{ixl}$, $T_{ixn}$) depending on the number of acquired images using a MRF pulse sequence.

8. A magnetic resonance imaging (MRI) system (1) comprising:

    a magnet system (2) configured to generate a polarizing magnetic field about at least a portion of a subject arranged in the MRI system;
    a plurality of gradient coils (3, 4, 5) configured to apply a gradient field to the polarizing magnetic field;
    a radio frequency (RF) system (5) configured to apply an excitation field to a subject and acquire MR image data from a ROI;
    a storage medium providing a first magnetic resonance fingerprinting dictionary and a plurality of transformation matrices, the transformation matrices being designed to transform the first magnetic resonance fingerprinting dictionary to a second magnetic resonance fingerprinting dictionary, the second magnetic resonance fingerprinting dictionary having a second length being shorter than the first length of the first magnetic resonance fingerprinting dictionary;
    a computer system programmed to:

        control the plurality of gradient coils (2, 3, 4) and the RF system to acquire magnetic resonance fingerprinting (MRF) data from a subject;
        reconstruct an MRF time series of images from the MRF data;
        obtain signal evolutions out of the time series of images;
        obtain the second magnetic resonance fingerprinting dictionary;
        obtain MR parameters by matching the signal evolutions with the second magnetic resonance fingerprinting dictionary; generate MR parameter maps from the matched parameters.

9. The MR system of claim 8, wherein the size of the second magnetic resonance fingerprinting dictionary is less than 25% of the size of the first magnetic resonance fingerprinting dictionary.

10. The MR system of claim 8 or 9, wherein the computer program is programmed to choose a transformation matrix out of a plurality of transformation matrices depending on the number of images chosen in a MRF pulse sequence.

11. The MR system of one of claim 8-10, wherein the first magnetic resonance dictionary is compressed.

12. A non-transitory computer-readable storage medium storing computer executable instructions that when executed by a computer control the computer to perform a method for producing a second magnetic resonance fingerprinting dictionary, comprising:

providing a first magnetic resonance fingerprinting dictionary, the fingerprints of the magnetic resonance fingerprinting dictionary having a first length,
providing a plurality of transformation matrices, the transformation matrices at least being designed to shorten the fingerprints to a second length shorter than the first length,
choosing one of the transformation matrices depending on the number of acquired images using a MRF pulse sequence,

obtaining the second magnetic resonance fingerprinting dictionary by multiplying the first magnetic resonance fingerprinting dictionary with the transformation matrix, the fingerprints of the magnetic resonance fingerprinting dictionary having the second length.

13. The non-transitory computer-readable storage medium of claim 12, wherein the first magnetic resonance dictionary is compressed.

14. The non-transitory computer-readable storage medium of claim 13, further comprising the transformation matrix being designed to decompress the first magnetic resonance fingerprinting dictionary.

15. The non-transitory computer-readable storage medium of claim 12, 13, or 14, further comprising the transformation matrix being designed to compress the second magnetic resonance fingerprinting dictionary.

FIG 1

EP 3 572 825 A2

## FIG 2

200

```
        execute MRF pulse sequence      ~ 210
                    │
                    ▼
          generate image series         ~ 220
                    │
                    ▼
        determine signal evolutions     ~ 230
                    │
                    ▼
              ╱ does first  ╲
             ⟨ dictionary fit? ⟩
              ╲            ╱
        240    no          yes    270
        │                   │
        ▼                   ▼
  ┌──────────────┐   ┌──────────────┐
  │ create second│   │   compare    │
  │  dictionary  │   └──────────────┘
  └──────────────┘          │
        │                   ▼
        ▼          ┌──────────────────┐
  ┌──────────┐     │create parameter  │
  │ compare  │~250 │      maps        │
  └──────────┘     └──────────────────┘
        │                   280
        ▼
  ┌──────────────────┐
  │create parameter  │~260
  │      maps        │
  └──────────────────┘
```

## FIG 3

300

receive signal evolution y — 310

dictionary compressed?

320     yes     no     340

compress signal evolution y to yc

compare

compare — 330

## FIG 4

400

choose transformation matrix — 410

$$D_2 = T_{jxn} D_1$$ — 420

## FIG 5

500

choose transformation matrix — 510

$$D_2 = T_{jxl} Dc_1$$ — 520

# FIG 6

600

| choose transformation matrix | ~610 |

$$Dc_2 = T_{ixn}D_1$$ ~620

# FIG 7

700

| choose transformation matrix | ~710 |

$$Dc_2 = T_{ixl}Dc_1$$ ~720

# FIG 8

flip angle

800

810

image number

0  200  400  600

## FIG 9

flip angle

900

930

940

0    200    400    600    800    1000

image number

910    920    920

## FIG 10

$Dc_1$  $T_{200x200}$  $T_{300x200}$  $T_{400x200}$  $T_{500x200}$  $T_{600x200}$

$T_{700x200}$  $T_{800x200}$  $T_{900x200}$  $T_{1000x200}$ $T_{1100x200}$

$T_{1200x200}$ $T_{1300x200}$ $T_{1400x200}$ $T_{1500x200}$ $T_{1600x200}$

$T_{1700x200}$ $T_{1800x200}$ $T_{1900x200}$ $T_{2000x200}$ $T_{2100x200}$

...

7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **MA et al.** Magnetic Resonance Fingerprinting. *Nature,* 2013, vol. 495, 187-192

- **MCGIVNEY et al.** SVD Compression for Magnetic Resonance Fingerprinting in the Time Domain. *IEEE Transactions on Medical Imaging,* 2014, vol. 33 (12), 2311-2322